(19) Europäisches Patentamt
European Patent Office
Office européen des brevets

(11) Veröffentlichungsnummer: **0 073 880**
**A2**

(12) # EUROPÄISCHE PATENTANMELDUNG

(21) Anmeldenummer: 82104537.4

(22) Anmeldetag: 25.05.82

(51) Int. Cl.[3]: C 07 D 249/02
C 07 D 249/10, C 07 D 233/60
C 07 D 231/12, C 07 D 231/16
C 07 D 231/18, A 01 N 43/50
A 01 N 43/56, A 01 N 43/64

(30) Priorität: 04.06.81 DE 3122175
02.03.82 DE 3207484

(43) Veröffentlichungstag der Anmeldung:
16.03.83 Patentblatt 83/11

(84) Benannte Vertragsstaaten:
AT BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Förster, Heinz, Dr.
Am Eckbusch 47
D-5600 Wuppertal 1(DE)

(72) Erfinder: Eue, Ludwig, Dr.
Paul-Klee-Strasse 36
D-5090 Leverkusen 1(DE)

(72) Erfinder: Schmidt, Robert, Dr.
Im Waldwinkel 110
D-5060 Bergisch-Gladbach 2(DE)

(72) Erfinder: Lürssen, Klaus, Dr.
August-Kierspel-Strasse 89
D-5060 Bergisch-Gladbach 2(DE)

(54) **Substituierte Phenoxyphenoxyalkancarbonsäure-azolylalkylester, Verfahren zu ihrer Herstellung sowie ihre Verwendung als Herbizide und Pflanzenwuchsregulatoren.**

(57) Substituierte Phenoxyphenoxyalkancarbonsäure-azolylalkylester der Formel

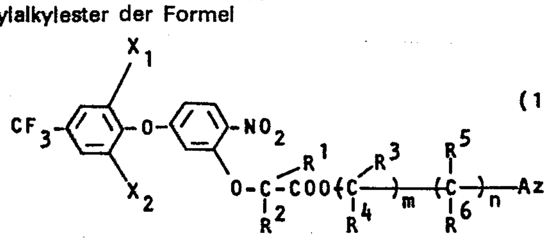

(I)

in welcher

$X^1$ und $X^2$ unabhängig voneinander für Wasserstoff oder Halogen stehen,

$R^1$, $R^2$, $R^3$, $R^4$, $R^5$ und $R^6$ unabhängig voneinander für Wasserstoff oder Alkyl mit 1 bis 4 Kohlenstoffatomen stehen,

m für 1 oder 2 steht,

n für 0 oder 1 steht und

Az für einen gegebenenfalls substituierten Azolylrest steht,

sowie deren Säureadditions-Salze, Metallsalz-Komplexe und quaternäre Alkylierungsmittel-Additionssalze, mehrere Verfahren zu deren Herstellung und deren Verwendung als Herbizide und Pflanzenwuchsregulatoren.

Halogenalkancarbonsäure-azolylalkylester der Formel

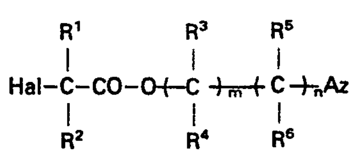

(V)

in welcher

$R^1$, $R^2$, $R^3$, $R^4$, $R^5$, $R^6$, Az, m und n die oben angegebene Bedeutung haben und

Hal für Chlor oder Brom steht,

und deren Herstellung.

Verbindungen der Formel

./...

EP 0 073 880 A2

in welcher
$X^1$, $X^2$, $R^1$, $R^2$, $R^3$, $R^4$, $R^5$, $R^6$, Az, m und n die oben angegebene Bedeutung haben,
und deren Herstellung.

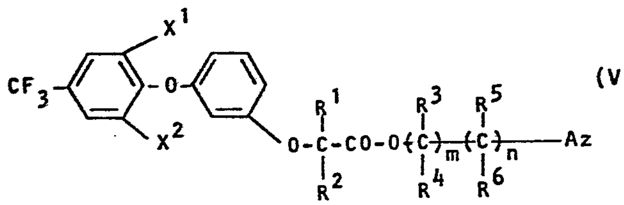

(V1)

0073880



BAYER AKTIENGESELLSCHAFT 5090 Leverkusen, Bayerwerk

Zentralbereich
Patente, Marken und Lizenzen Dü-by-c
Ib

Substituierte Phenoxyphenoxyalkancarbonsäure-azolyl-alkylester, Verfahren zu ihrer Herstellung sowie ihre Verwendung als Herbizide und Pflanzenwuchsregulatoren

Die Erfindung betrifft neue substituierte Phenoxyphenoxyalkancarbonsäure-azolylalkylester, mehrere Verfahren zu deren Herstellung sowie deren Verwendung als Herbizide und Pflanzenwuchsregulatoren.

Es ist bereits bekannt geworden, daß bestimmte Phenoxyphenoxyalkancarbonsäureester herbizide Eigenschaften besitzen (vgl. DE-OS 2 311 638 und US-PS 4 093 446). So läßt sich zum Beispiel α-(5-(2-Chlor-4-trifluormethyl-phenoxy)-2-nitro-phenoxy)-propionsäureethylester zur Bekämpfung von Unkraut verwenden. Die Wirkung dieses Stoffes ist gut, jedoch ist die Selektivität nicht immer ausreichend.

Es wurden nun neue substituierte Phenoxyphenoxyalkancarbonsäure-azolylalkylester der Formel

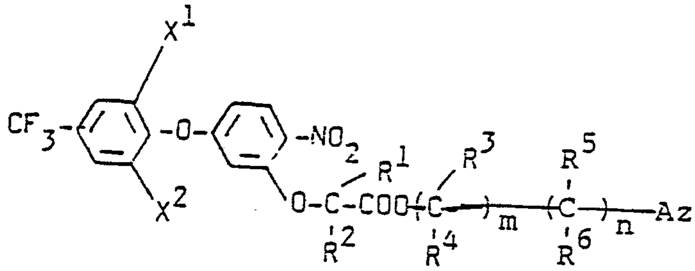

(I)

in welcher

$X^1$ und $X^2$ unabhängig voneinander für Wasserstoff oder Halogen stehen,

| | |
|---|---|
| $R^1$,$R^2$,$R^3$,$R^4$,$R^5$ und $R^6$ | unabhängig voneinander für Wasserstoff oder Alkyl mit 1 bis 4 Kohlenstoffatomen stehen, |
| m | für 1 oder 2 steht, |
| n | für 0 oder 1 steht und |
| Az | für einen gegebenenfalls substituierten Azolylrest steht, |

sowie deren Säureadditions-Salze Metallsalzkomplexe und quaternäre Alkylierungsmittel-Additionssalze

gefunden.

Weiterhin wurde gefunden, daß man die neuen substituierten Phenoxyphenoxyalkancarbonsäure-azolylalkylester der Formel (I), deren Säureadditions-Salze, Metallsalz-Komplexe und quaternäre Alkylierungsmittel-Additionssalze erhält, wenn man

a) Phenoxyphenoxyalkancarbonsäurechloride der Formel

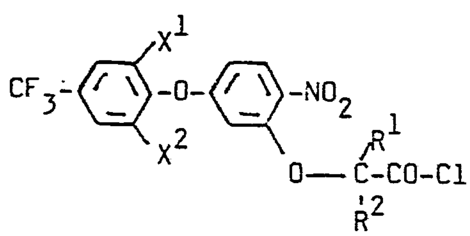

(II)

in welcher

$X^1$, $X^2$, $R^1$ und $R^2$ die oben angegebene Bedeutung haben,

mit Hydroxylalkylazolen der Formel

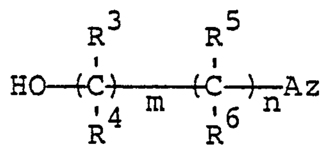

(III)

in welcher

$R^3$, $R^4$, $R^5$, $R^6$, Az, n und m die oben angegebene Bedeutung haben,

gegebenenfalls in Gegenwart eines Säureakzeptors und gegebenenfalls in Gegenwart eines Verdünnungsmittels umsetzt, oder wenn man

b) Phenoxyphenole der Formel

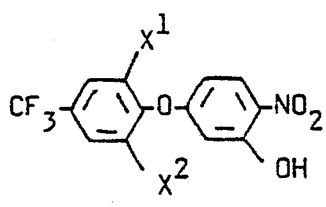

(IV)

in welcher

$X^1$ und $X^2$ die oben angegebene Bedeutung haben

mit Halogenalkancarbonsäure-azolylalkylestern der Formel

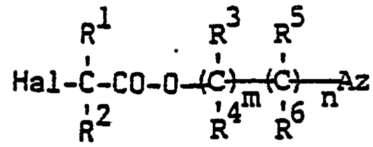 (V)

in welcher

$R^1, R^2, R^3, R^4, R^5, R^6$, Az, m und n die oben angegebene Bedeutung haben und

Hal für Chlor oder Brom steht,

gegebenenfalls in Gegenwart eines Säureakzeptors und gegebenenfalls in Gegenwart eines Verdünnungsmittels umsetzt, oder wenn man

c) Phenoxyphenoxyalkancarbonsäure-azolylalkylester der Formel

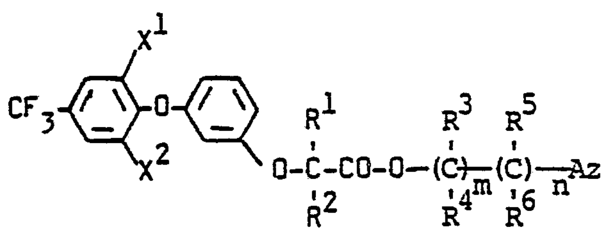 (VI)

in welcher

$X^1, X^2, R^1, R^2, R^3, R^4, R^5, R^6$, Az, m und n die oben angegebene Bedeutung haben,

mit Salpetersäure gegebenenfalls in Gegenwart eines Katalysators und gegebenenfalls in Gegenwart eines Verdünnungsmittels umsetzt,

und gegebenenfalls anschließend an die so erhaltenen Phenoxyphenoxyalkancarbonsäure-azolylalkylester der Formel (I) eine Säure, ein Metallsalz oder ein Alkylierungsmittel addiert.

Schließlich wurde gefunden, daß sich die neuen substituierten Phenoxyphenoxyalkancarbonsäure-azolylalkylester der Formel (I) sowie deren Säureadditions-Salze, Metallsalz-Komplexe und quaternäre Alkylierungsmittel-Additionssalze durch hervorragende herbizide und pflanzenwuchsregulierende Eigenschaften auszeichnen.

Überraschenderweise besitzen die erfindungsgemäßen substituierten Phenoxyphenoxyalkancarbonsäure-azolylalkylester der Formel (I) sowie deren Säureadditions-Salze, Metallsalz-Komplexe und quaternäre Alkylierungsmittel-Additionssalze eine wesentlich bessere selektive herbizide Wirksamkeit als der α-(5-(2-Chlor-4-trifluormethyl-phenoxy)-2-nitro-phenoxy)-propionsäureethylester, welches ein konstitutionell ähnlicher vorbekannter Wirkstoff gleicher Wirkungsrichtung ist. Darüber hinaus lassen sich die erfindungsgemäßen Stoffe auch als Pflanzenwuchsregulatoren einsetzen. Vor allem können sie als Defoliants und Desiccants bei Baumwolle verwendet werden.

Die erfindungsgemäßen substituierten Phenoxyphenoxyalkancarbonsäure-azolylalkylester sind durch die

Formel (I) allgemein definiert. In dieser Formel stehen vorzugsweise

$X^1$ für Chlor,
$X^2$ für Wasserstoff oder Chlor,
$R^1$,$R^2$,$R^3$,$R^4$,$R^5$ und $R^6$ unabhängig voneinander für Wasserstoff oder Methyl,
m für 1 oder 2,
n für 0 oder 1 und

Az für einen über ein Ring-Stickstoffatom gebundenen Pyrazolyl-, Imidazolyl-, 1,2,4-Triazolyl-, 1,2,3-Triazolyl- oder 1,3,4-Triazolyl-Rest, wobei jeder dieser Azolyl-Reste ein- oder mehrfach, gleichartig oder verschieden substituiert sein kann durch Fluor, Chlor, Brom, Jod, Alkyl mit 1 bis 4 Kohlenstoffatomen, welches seinerseits gegebenenfalls durch Fluor, Chlor, Cyano, Alkoxy mit 1 bis 4 Kohlenstoffatomen, Alkylthio mit 1 bis 4 Kohlenstoffatomen, Alkylsulfinyl mit 1 bis 4 Kohlenstoffatomen, Alkylsulfonyl mit 1 bis 4 Kohlenstoffatomen und/oder Alkoxycarbonyl mit 1 bis 4 Kohlenstoffatomen in der Alkoxygruppe substituiert ist, Alkoxy mit 1 bis 4 Kohlenstoffatomen, Alkylthio mit 1 bis 4 Kohlenstoffatomen, Cyano, Carboxy, Alkoxycarbonyl mit 1 bis 4 Kohlenstoffatomen in der Alkoxygruppe, Alkylcarbonyl mit 1 bis 4 Kohlenstoffatomen in der Alkylgruppe und/oder Phenyl, welches seinerseits gegebenenfalls durch Halogen, Alkyl mit 1 bis 4 Kohlenstoffatomen, Trifluormethyl, Cyano, Nitro und/oder Alkoxy mit 1 bis 4 Kohlenstoffatomen substituiert ist.

Besonders bevorzugt sind diejenigen erfindungsgemäßen substituierten Phenoxyphenoxyalkancarbonsäure-azolylalkylester der Formel (I), in denen

$X^1$ für Chlor steht,

$X^2$ für Wasserstoff oder Chlor steht,

$R^1$ für Methyl steht,

$R^2$,$R^3$ und $R^4$ für Wasserstoff stehen,

$R^5$ und $R^6$ für Wasserstoff oder Methyl stehen,

m für 1 oder 2 steht, n für 0 oder 1 steht und

Az für einen über ein Ring-Stickstoffatom gebundenen Pyrazolyl-, Imidazolyl-, 1,2,4-Triazolyl-, 1,2,3-Triazolyl- oder 1,3,4-Triazolyl-Rest steht, wobei jeder dieser Azolyl-Reste ein-, zwei- oder dreifach, gleichartig oder verschieden substituiert sein kann durch Fluor, Chlor, Brom, Alkyl mit 1 oder 2 Kohlenstoffatomen, welches seinerseits gegebenenfalls durch Fluor, Chlor, Cyano, Alkoxy mit 1 oder 2 Kohlenstoffatomen, Alkylthio mit 1 oder 2 Kohlenstoffatomen, Alkylsulfinyl mit 1 oder 2 Kohlenstoffatomen, Alkylsulfonyl mit 1 oder 2 Kohlenstoffatomen und/oder Alkoxycarbonyl mit 1 oder 2 Kohlenstoffatomen in der Alkoxygruppe substituiert ist, Alkoxy mit 1 oder 2 Kohlenstoffatomen, Alkylthio mit 1 oder 2 Kohlenstoffatomen, Cyano, Carboxy, Alkoxycarbonyl mit 1 oder 2 Kohlenstoffatomen in der Alkoxygruppe, Alkylcarbonyl mit 1 oder 2 Kohlenstoffatomen in der Alkylgruppe und/oder Phenyl, welches seinerseits gegebenenfalls durch Fluor, Chlor, Brom, Alkyl mit 1 oder 2 Kohlenstoffatomen, Trifluormethyl, Cyano, Nitro und/oder Alkoxy mit 1 oder 2 Kohlenstoffatomen substituiert ist.

Bevorzugt sind außerdem Säureadditions-Salze von Phenoxyphenoxyalkancarbonsäure-azolylalkylestern der Formel (I), in denen $X^1,X^2,R^1,R^2,R^3,R^4,R^5,R^6$,Az,m und n die vorzugsweise genannten Bedeutungen haben. Besonders bevorzugt sind dabei solche Säureadditions-Salze, die durch Addition von Halogenwasserstoffsäure, wie zum Beispiel Chlorwasserstoffsäure und Bromwasserstoffsäure, insbesondere Chlorwasserstoffsäure, ferner Phosphorsäure, Salpetersäure, Schwefelsäure, mono- und bifunktionelle Carbonsäuren und Hydroxycarbonsäuren, wie zum Beispiel Essigsäure, Maleinsäure, Bernsteinsäure, Fumarsäure, Weinsäure, Zitronensäure, Salicylsäure, Sorbinsäure, Milchsäure, sowie Sulfonsäuren, wie zum Beispiel p-Toluolsulfonsäure und 1,5-Naphthalindisulfonsäure entstehen.

Bevorzugt sind auch Metallsalzkomplexe von Phenoxyphenoxyalkancarbonsäure-azolylalkylestern der Formel (I), in denen $X^1,X^2,R^1,R^2,R^3,R^4,R^5,R^6$,Az, m und n die vorzugsweise genannten Bedeutungen haben. Besonders bevorzugt sind dabei solche Metallsalz-Komplexe, die als Kationen Metalle der II. bis IV. Hauptgruppe oder der I. und II. oder IV. bis VIII. Nebengruppe des Periodensystems der Elemente enthalten, wobei Kupfer, Zink, Mangan, Magnesium, Zinn, Eisen und Nickel beispielhaft genannt seien.

Anionen dieser Metallsalz-Komplexe sind vorzugsweise solche, die sich von Halogenwasserstoffsäuren, insbesondere Chlorwasserstoffsäure und Bromwasserstoffsäure, ferner von Phosphorsäure, Salpetersäure und Schwefelsäure ableiten.

Bevorzugt sind schließlich auch quaternäre Alkylierungsmittel-Additionssalze von Phenoxy-phenoxyalkancarbonsäure-azolylalkylestern der Formel (I), in denen $X^1$, $X^2$, $R^1$, $R^2$, $R^3$, $R^4$, $R^5$, $R^6$, Az, m und n die vorzugsweise genannten Bedeutungen haben. Besonders bevorzugt sind dabei solche quaternären Salze, die durch Addition von Alkylchloriden, -bromiden und -iodiden mit jeweils 1 bis 4 Kohlenstoffatomen bzw. von Dialkylsulfaten mit 1 bis 4 Kohlenstoffatomen je Alkylgruppe entstehen.

Verwendet man α-(5-(2-Chlor-4-trifluormethylphenoxy)-2-nitro-phenoxy)-propionsäurechlorid und 1-Hydroxymethyl-pyrazol als Ausgangsstoffe, so läßt sich der Verlauf des erfindungsgemäßen Verfahrens (a) durch das folgende Formelschema wiedergeben:

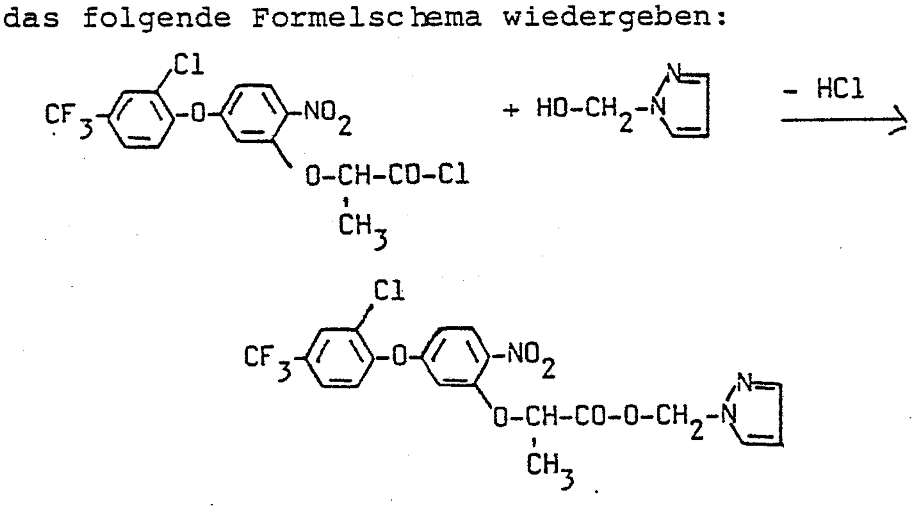


Verwendet man 5-(2,6-Dichlor-4-trifluormethyl-phenoxy)-2-nitro-phenol und α-Brom-propionsäure-pyrazolyl-methylester als Ausgangsstoffe, so läßt sich der Verlauf des erfindungsgemäßen Verfahrens (b) durch das folgende Formelschema wiedergeben:

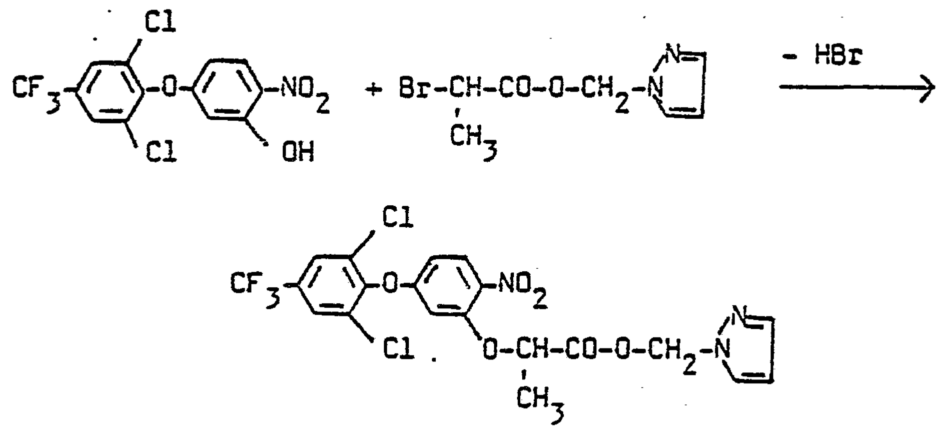


Verwendet man α-(3-(2-Chlor-4-trifluormethyl-phenoxy)-phenoxy)-propionsäure-pyrazolyl-methylester als Ausgangsstoff und Salpetersäure als Nitrierungsmittel, so läßt sich der Verlauf des erfindungsgemäßen Verfahrens (c) durch das folgende Formelschema wiedergeben:

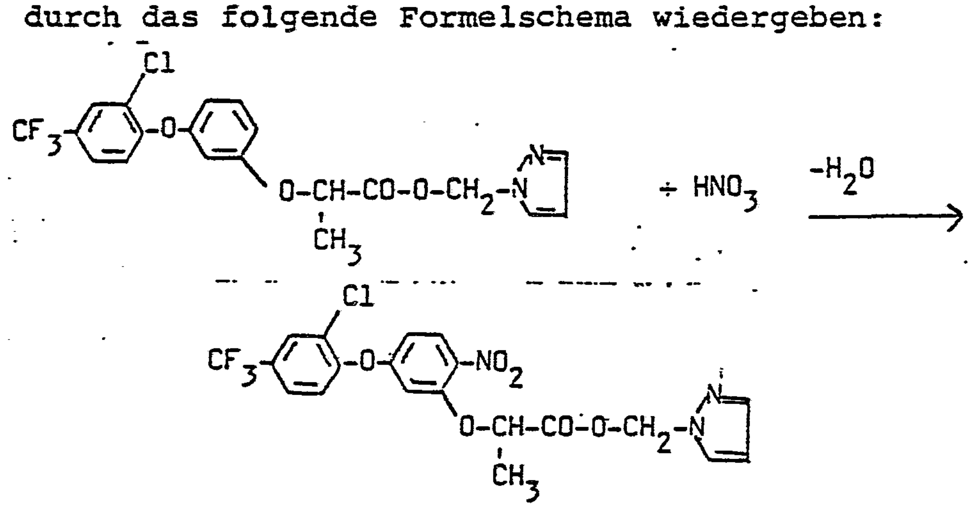


Die bei dem erfindungsgemäßen Verfahren (a) als Ausgangsstoffe benötigten Phenoxyphenoxyalkancarbonsäurechloride sind durch die Formel (II) allgemein definiert. In dieser Formel haben $X^1$, $X^2$, $R^1$ und $R^2$

vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele seien 5-(2-Chlor-4-trifluormethyl-phenoxy)-2-nitro-phenoxy-essigsäurechlorid, 5-(2,6-Dichlor-4-trifluormethyl-phenoxy)-2-nitro-phenoxy-essigsäurechlorid, α-(5-(2-Chlor-4-trifluormethyl-phenoxy)-2-nitro-phenoxy)-propionsäurechlorid und α-(5-(2,6-Dichlor-4-trifluormethyl-phenoxy)-2-nitro-phenoxy)-propionsäurechlorid genannt.

Die Verbindungen der Formel (II) sind bereits bekannt (vgl. DE-OS 2 906 237).

Die bei dem Verfahren (a) weiterhin als Ausgangsstoffe zu verwendenden Hydroxyalkylazole sind durch die Formel (III) definiert. In dieser Formel haben $R^3$, $R^4$, $R^5$, $R^6$, Az, m und n vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele für Hydroxyalkylazole der Formel (III) seien im einzelnen genannt:
1-Hydroxymethyl-pyrazol, 1-(1-Hydroxyethyl)-pyrazol, 1-Hydroxymethylimidazol, 1-(1-Hydroxyethyl)-imidazol, 1-Hydroxymethyl-1,2,4-triazol, 1-(1-Hydroxyethyl)-1,2,4-triazol, 1-Hydroxymethyl-1,3,4-triazol, 1-(1-Hydroxyethyl)-1,3,4-triazol, 1-Hydroxymethyl-3,5-dimethyl-pyrazol, 1-Hydroxymethyl-2-methyl-imidazol, 1-Hydroxymethyl-3-methyl-pyrazol, 3-Chlor-1-hydroxymethyl-1,2,4-triazol, 4-Chlor-1-hydroxymethyl-pyrazol, 1-Hydroxymethyl-4-methyl-pyrazol, 1-Hydroxymethyl-4-methoxy-pyrazol, 1-Hydroxymethyl-3-methylthio-pyrazol, 4-Chlor-1-hydroxymethyl-3,5-dimethyl-pyrazol, 1-(2-Hydroxyethyl)-pyrazol, 1-(2-Hydroxyethyl)-imidazol und 1-(Hydroxy-1,1-dimethyl-ethyl)-1,2,4-triazol.

Die Verbindungen der Formel (III) sind bereits bekannt (vgl. DE-OS 2 835 157 und 2 835 158).

Die bei dem erfindungsgemäßen Verfahren (b) als Ausgangsstoffe benötigten Phenoxyphenole sind durch die Formel (IV) allgemein definiert. In dieser Formel haben $X^1$ und $X^2$ vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele seien 5-(2-Chlor-4-trifluormethyl-phenoxy)-2-nitro-phenol und 5-(2,6-Dichlor-4-trifluormethyl-phenoxy)-2-nitro-phenol genannt.

Die Verbindungen der Formel (IV) sind bereits bekannt (vgl. US-PS 3 928 416).

Die bei dem Verfahren (b) weiterhin als Ausgangsstoffe zu verwendenden Halogenalkancarbonsäure-azolylalkylester sind durch die Formel (V) allgemein definiert.

In dieser Formel haben $R^1$,$R^2$,$R^3$,$R^4$,$R^5$,$R^6$,Az, m und n vorzugsweise diejenigen Bedeutungen, die bereits im Zusammenhang mit der Beschreibung der erfindungsgemäßen Stoffe der Formel (I) vorzugsweise für diese Reste genannt wurden. Hal steht in der Formel (V) für Chlor oder Brom.

Als Beispiele für die Verbindungen der Formel (V) seien genannt: Bromessigsäure-, Chloressigsäure-, α-Brompropionsäure- und α-Chlor-propionsäure-pyrazol-1-yl-methylester, -(1-pyrazol-1-yl)-ethylester, -imidazol-1-yl-methylester, -1,2,4-triazol-1-yl-methylester, -1,3,4-triazol-1-yl-methylester, -(3,5-dimethyl-pyrazol-1-yl)-methylester, -(2-methyl-imidazol-1-yl)-methylester, -(3-methyl-pyrazol-1-yl)-methylester, -(3-chlor-1,2,4-triazol-1-yl)-methylester, -(4-chlor-pyrazol-1-yl)-methylester, -(4-methyl-pyrazol-1-yl)-methylester, -(4-methoxypyrazol-1-yl)-methylester, -(3-methylthio-pyrazol-1-yl)-methylester und -(4-chlor-3,5-dimethyl-pyrazol-1-yl)-methylester.

Die Verbindungen der Formel (V) sind noch nicht in der Literatur beschrieben. Sie lassen sich jedoch nach an sich bekannten Verfahren in einfacher Weise herstellen. Man erhält sie beispielsweise, wenn man Halogencarbonsäurehalogenide der Formel

$$\text{Hal}-\overset{\displaystyle R^1}{\underset{\displaystyle R^2}{\overset{|}{\underset{|}{C}}}}-\text{CO}-\text{Hal}' \qquad \text{(VII)}$$

in welcher

$R^1$ und $R^2$ die oben angegebene Bedeutung haben und

Hal sowie Hal' für Chlor oder Brom stehen,

mit Hydroxyalkylazolen der Formel

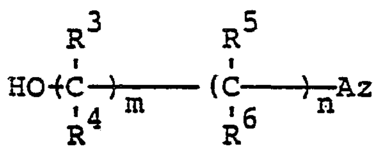

(III)

in welcher

$R^3$,$R^4$,$R^5$,$R^6$,Az, m und n die oben angegebenen Bedeutungen haben,

gegebenenfalls in Gegenwart eines Säureakzeptors und gegebenenfalls in Gegenwart eines Verdünnungsmittels umsetzt.

Die bei dem obigen Verfahren als Ausgangsstoffe benötigten Halogencarbonsäurehalogenide sind durch die Formel (VII) definiert. In dieser Formel haben $R^1$ und $R^2$ vorzugsweise diejenigen Bedeutungen, die bereits im Zusammenhang mit der Beschreibung der erfindungsgemäßen Stoffe der Formel (I) vorzugsweise für diese Reste genannt wurden. Hal und Hal' stehen für Chlor oder Brom.

Als Beispiele für die Verbindungen der Formel (VII) seien Chloracetylchlorid, Bromacetylchlorid, Bromacetylbromid, α-Chlor-propionsäure-chlorid, α-Brompropionsäurechlorid und α-Brom-propionsäurebromid genannt.

Die Verbindungen der Formel (VII) sind bekannt.

Bei dem obigen Verfahren entsprechen die Umsetzungsbedingungen im einzelnen denjenigen des erfindungsgemäßen Verfahrens (a).

Die bei dem erfindungsgemäßen Verfahren (c) als Ausgangsstoffe benötigten Phenoxyphenoxy-alkancarbonsäure-azolylalkylester sind durch die Formel (VI) allgemein definiert. In dieser Formel haben $X^1$,$X^2$,$R^1$, $R^2$,$R^3$,$R^4$,$R^5$,$R^6$,Az,m und n vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele für die Verbindungen der Formel (VI) seien genannt: 3-(2-Chlor-4-trifluormethyl-phenoxy)- und 3-(2,6-Dichlor-4-trifluormethyl-phenoxy)-phenoxyessigsäure- und - α -phenoxy-propionsäure-pyrazol-1-yl-methylester, -(1-pyrazol-1-yl)-ethylester, -imidazol-1-yl-methylester, -1,2,4-triazol-1-yl-methylester, -1,3,4-triazol-1-yl-methylester, -(3,5-dimethyl-pyrazol-1-yl)-methylester, -(2-methyl-imidazol-1-yl)-methylester, -(3-methyl-

pyrazol-1-yl)-methylester, -(3-chlor-1,2,4-triazol-1-yl)-methylester, -(4-chlor-pyrazol-1-yl)-methylester, -(4-methyl-pyrazol-1-yl)-methylester, -(4-methoxy-pyrazol-1-yl)-methylester, -(3-methylthio-pyrazol-1-yl)-methylester und -(4-chlor-3,5-dimethyl-pyrazol-1-yl)-methylester.

Die Verbindungen der Formel (VI) sind noch nicht in der Literatur beschrieben; sie lassen sich jedoch nach an sich bekannten Verfahren in einfacher Weise herstellen. Man erhält sie beispielsweise, wenn man Phenoxyphenole der Formel

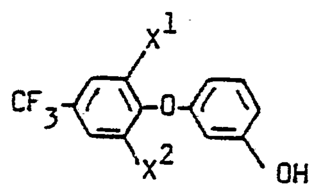

(VIII)

in welcher

$X^1$ und $X^2$ die oben angegebene Bedeutung haben,

mit Halogenalkancarbonsäure-azolylalkylestern der Formel

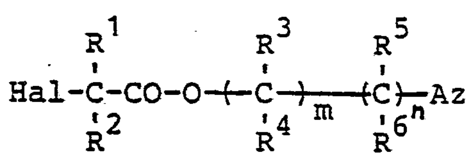

(V)

in welcher

$R^1$, $R^2$, $R^3$, $R^4$, $R^5$, $R^6$, Az, m, n und Hal die oben angegebene Bedeutung haben,

gegebenenfalls in Gegenwart eines Säureakzeptors und gegebenenfalls in Gegenwart eines Verdünnungsmittels umsetzt.

Die bei dem obigen Verfahren als Ausgangsstoffe benötigten Phenoxyphenole sind durch die Formel (VIII) definiert. In dieser Formel haben $X^1$ und $X^2$ vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele für die Verbindungen der Formel (VIII) seien 3-(2-chlor-4-trifluormethyl-phenoxy)-phenol und 3-(2,6-Dichlor-4-trifluormethyl-phenoxy)-phenol genannt.

Die Verbindungen der Formel (VIII) sind bereits bekannt (vgl. DE-OS 2 805 981).

Bei dem obigen Verfahren entsprechen die Umsetzungsbedingungen im einzelnen denjenigen des erfindungsgemäßen Verfahrens (b).

Das erfindungsgemäße Verfahren (a) wird vorzugsweise in Gegenwart eines Verdünnungsmittels durchgeführt.

Als derartige Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methyl-isobutylketon, außerdem Ester, wie Essigsäure-methylester und -ethylester, ferner Nitrile, wie z.B. Acetonitril und Propionitril, darüber hinaus Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Als Säureakzeptoren können bei dem erfindungsgemäßen Verfahren (a) alle üblichen Säurebindemittel verwendet werden. Hierzu gehören vorzugsweise Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin und Pyridin.

Die Reaktionstemperaturen können bei dem erfindungsgemäßen Verfahren (a) innerhalb eines größeren Bereiches

variiert werden. Im allgemeinen arbeitet man bei Temperaturen zwischen -20 und +100°C, vorzugsweise zwischen 0 und 80°C.

Das erfindungsgemäße Verfahren (a) wird im allgemeinen unter Normaldruck durchgeführt.

Bei der Durchführung des erfindungsgemäßen Verfahrens (a) setzt man auf 1 Mol Phenoxyphenoxyalkancarbonsäurechlorid der Formel (II) im allgemeinen 0,9 bis 1,5 Mol, vorzugsweise 1,0 bis 1,3 Mol Hydroxyalkylazol der Formel (III) und gegebenenfalls 1 bis 1,5 Mol, vorzugsweise 1,05 bis 1,3 Mol Säureakzeptor ein. Wegen des leicht exothermen Reaktionsverlaufs werden die Ausgangskomponenten vorzugsweise unter Eiskühlung zusammengegeben und dann bei etwas höherer Temperatur bis zum Ende der Umsetzung gerührt. Die Aufarbeitung erfolgt nach üblichen Methoden. Es wird beispielsweise mit Toluol verdünnt, mit Wasser gewaschen, getrocknet, filtriert und eingeengt. Die hierbei zurückbleibenden Produkte werden durch ihren Schmelzpunkt charakterisiert.

Das erfindungsgemäße Verfahren (b) wird vorzugsweise in Gegenwart von Verdünnungsmitteln durchgeführt. Als Verdünnungsmittel kommen hierbei vorzugsweise alle diejenigen Solventien in Betracht, die bereits im Zusammenhang mit der Beschreibung des erfindungsgemäßen Verfahrens (a) vorzugsweise genannt wurden.

Das erfindungsgemäße Verfahren (b) wird vorzugsweise in Gegenwart von Säureakzeptoren durchgeführt. Als Säureakzeptoren kommen hierbei vorzugsweise diejenigen Säurebindemittel in Betracht, die bereits im Zusammenhang mit der Beschreibung des erfindungsgemäßen Verfahrens (a) vorzugsweise genannt wurden.

Die Reaktionstemperaturen können auch im Falle des erfindungsgemäßen Verfahrens (b) in einem größeren Bereich variiert werden. Im allgemeinen arbeitet man bei Temperaturen zwischen 0 und 100°C, vorzugsweise zwischen 10 und 80°C.

Das erfindungsgemäße Verfahren (b) wird im allgemeinen unter Normaldruck durchgeführt.

Bei der Durchführung des erfindungsgemäßen Verfahrens (b) setzt man auf 1 Mol Phenoxyphenol der Formel (IV) im allgemeinen 0,9 bis 1,5 Mol, vorzugsweise 1,0 bis 1,3 Mol Halogenalkancarbonsäure-azolylalkylester der Formel (V) und gegebenenfalls 1 bis 1,5 Mol, vorzugsweise 1,05 bis 1,3 Mol Säureakzeptor ein.

Die Ausgangsstoffe werden bei Raumtemperatur zusammengegeben und gegebenenfalls bei leicht erhöhter Temperatur bis zum Ende der Umsetzung gerührt. Die Aufarbeitung wird nach üblichen Methoden, beispielsweise wie oben beschrieben, durchgeführt.

Das erfindungsgemäße Verfahren (c) wird gegebenenfalls unter Verwendung eines Verdünnungsmittels durchgeführt. Als Verdünnungsmittel kommen hierbei vorzugsweise halogenierte Kohlenwasserstoffe, wie z.B. Methylenchlorid, in Betracht.

Das erfindungsgemäße Verfahren (c) wird gegebenenfalls in Gegenwart eines Katalysators durchgeführt. Als Katalysatoren kommen vorzugsweise Protonensäuren, wie z.B. Schwefelsäure oder Essigsäure, in Betracht.

Die Reaktionstemperatur wird bei Verfahren (c) im allgemeinen zwischen -20 und +80°C, vorzugsweise zwischen 0 und 50°C gehalten. Verfahren (c) wird im allgemeinen bei Normaldruck durchgeführt.

Zur Durchführung des erfindungsgemäßen Verfahrens (c) setzt man auf 1 Mol Phenoxyphenoxyalkancarbonsäure-azolylalkylester der Formel (VI) im allgemeinen 0,9 bis 2 Mol, vorzugsweise 1,0 bis 1,3 Mol Salpetersäure und gegebenenfalls etwa die gleiche Menge eines Katalysators ein. Die Ausgangskomponenten werden vorzugsweise unter Eiskühlung zusammengegeben und dann gegebenenfalls bei leicht erhöhter Temperatur bis zum Ende der Umsetzung gerührt.

Die Aufarbeitung wird nach üblichen Methoden, beispielsweise durch Eingießen in Eiswasser, Absaugen und gegebenenfalls Umkristallisieren, durchgeführt.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen von Verbindungen der Formel (I) kommen vorzugsweise Salze von denjenigen Anionen und Kationen in Betracht, die bereits im Zusammenhang mit der Beschreibung der erfindungsgemäßen Metallsalz-Komplexe als bevorzugt genannt wurden.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren, Isolieren und gegebenenfalls durch Umkristallisation reinigen.

Zur Herstellung von quaternären Alkylierungsmittel-Additionssalzen von Verbindungen der Formel (I) kommen vorzugsweise diejenigen Alkylchloride, -bromide, -iodide und Alkylsulfate in Betracht, die bereits im Zusammenhang mit der Beschreibung der erfindungsgemäßen Stoffe vorzugsweise als Alkylierungsmittel genannt wurden.

Die quaternären Alkylierungsmittel-Additionssalze der Verbindungen der Formel (I) können in einfacher Weise unter den für derartige Alkylierungen üblichen Bedingungen hergestellt werden. So erhält man die betreffenden quaternären Salze zum Beispiel dadurch, daß man eine Verbindung der Formel (I) in einem inerten organischen Solvens, wie zum Beispiel Acetonitril, löst und das Alkylierungsmittel hinzugibt. Die quaternären Salze, die kristallin anfallen, lassen sich nach üblichen Methoden isolieren. Im allgemeinen werden die Stoffe durch Abfiltrieren isoliert und gegebenenfalls durch Waschen mit einem inerten organischen Lösungsmittel gereinigt.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine,

Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe sind besonders gut zur selektiven Unkrautbekämpfung in verschiedenen Kulturen, wie zum Beispiel Mais, Sojabohnen, Baumwolle und Getreide, geeignet.

Die erfindungsgemäßen Wirkstoffe besitzen außerdem eine sehr gute pflanzenwachstumsregulierende Wirksamkeit. Sie eignen sich besonders gut zur Wuchshemmung, sowie zur Entlaubung und Austrocknung der Blätter bei Baumwolle.

Die Wirkstoffe können in die üblichen Formulierungen überführt werden, wie Lösungen, Emulsionen, Spritzpulver, Suspensionen, Pulver, Stäubemittel, lösliche Pulver, Granulate, Suspensions-Emulsionskonzentrate, Wirkstoffimprägnierte Natur- und synthetische Stoffe, Feinstverkapselungen in polymeren Stoffen und Aerosole.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder

Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid, als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen oder organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden oder Pflanzenwachstumsregulatoren zur Unkrautbekämpfung bzw. als Pflanzenwuchsregulatoren Verwendung finden, wobei Fertigformulierungen oder Tankmischungen möglich sind. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen

bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können bei einer Anwendung als Herbizide sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Für die Anwendung der erfindungsgemäßen Wirkstoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Die angewandte Wirkstoffmenge kann in einem größeren Bereich schwanken. Sie hängt im wesentlichen ab von der Art des gewünschten Effektes. Im allgemeinen liegen die Aufwandmengen zwischen 0,01 und 50 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele

Beispiel 1

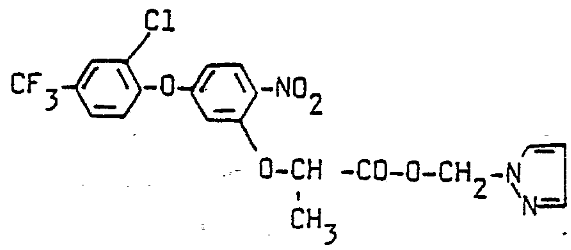

Eine Lösung von 21,2 g (0,05 Mol) α-(5-(2-Chlor-4-trifluormethylphenoxy)-2-nitro-phenoxy)-propionsäurechlorid in 30 ml Toluol wird zu einer auf 0 bis 5°C gekühlten Mischung aus 5,4 g (0,055 Mol) 1-Hydroxymethyl-pyrazol, 6 g (0,06 Mol) Triethylamin und 70 ml Toluol gegeben. Das Reaktionsgemisch wird über Nacht (ca. 15 Stunden) bei Raumtemperatur (ca. 20°C) gerührt. Zur Aufarbeitung wird mit Toluol verdünnt, dann mit Wasser, verdünnter Natronlauge und wieder mit Wasser gewaschen, getrocknet, filtriert und eingeengt. Man erhält 17,0 g (70 % der Theorie) α-(5-(2-Chlor-4-trifluormethyl-phenoxy)-2-nitrophenoxy)-propionsäure-pyrazol-1-yl-methylester in Form beigefarbener Kristalle vom Schmelzpunkt 75°C.

Nach der im Beispiel 1 beschriebenen Methode oder auch nach den Verfahrensvarianten (b) und (c) werden die in den nachstehenden Tabellen formelmäßig aufgeführten Verbindungen hergestellt.

Tabelle 1

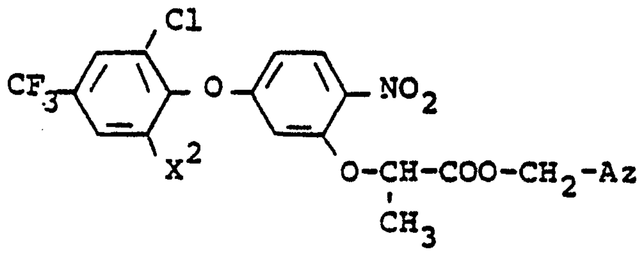

(Ia)

| Beispiel Nr. | $X^2$ | Az | Schmelzpunkt °C |
|---|---|---|---|
| 2 | Cl | 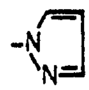 | 124 |
| 3 | H | 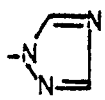 | |
| 4 | Cl | 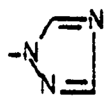 | |
| 5 | H | 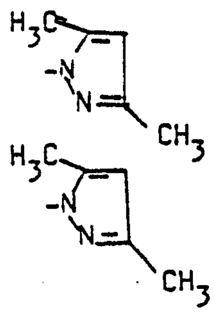 | |
| 6 | Cl | | |
| 7 | H | 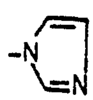 | |
| 8 | Cl | 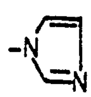 | |
| 9 | H | 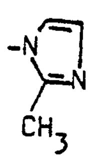 | |

Tabelle 1 (Fortsetzung)

| Beispiel Nr. | $X^2$ | Az | Schmelzpunkt °C |
|---|---|---|---|
| 10 | Cl | 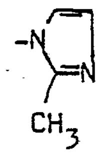 | |
| 11 | H | 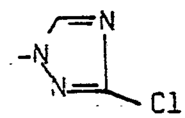 | |
| 12 | Cl | 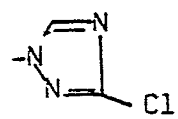 | |
| 13 | H | 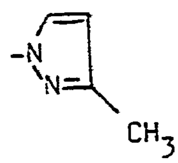 | |
| 14 | Cl | 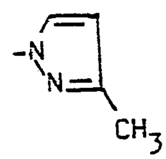 | |
| 15 | H | 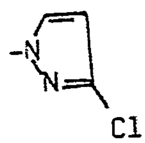 | |
| 16 | Cl | 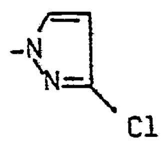 | |
| 17 | H | 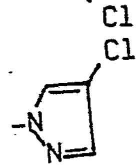 | |

Tabelle 1 (Fortsetzung)

| Beispiel Nr. | $X^2$ | Az | Schmelzpunkt °C |
|---|---|---|---|
| 18 | Cl | 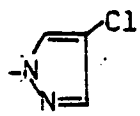 | |
| 19 | H | 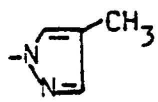 | |
| 20 | Cl | 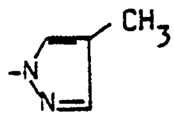 | |
| 21 | H | 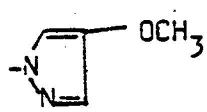 | |
| 22 | Cl | 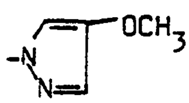 | |
| 23 | H | 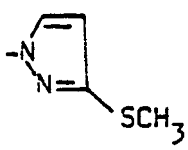 | |
| 24 | Cl | 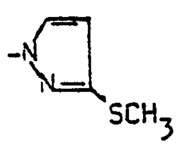 | |
| 25 | H | 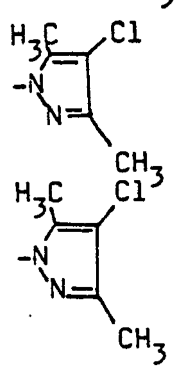 | |
| 26 | Cl | | |

Tabelle 2

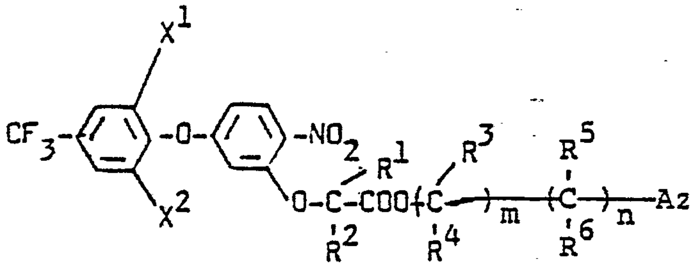

(I)

| Bsp. Nr. | $X^1$ | $X^2$ | $R^1$ | $R^2$ | $R^3$ | $R^4$ | $R^5$ | $R^6$ | n | m | Az | Schmelzpunkt bzw. Brechungsindex |
|---|---|---|---|---|---|---|---|---|---|---|---|---|
| 27 | Cl | Cl | H | $CH_3$ | H | H | - | - | 0 | 2 | 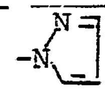 | 116 °C |
| 28 | Cl | H | H | $CH_3$ | H | H | - | - | 0 | 2 | 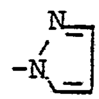 | $n_D^{20}$=1,5528 |
| 29 | Cl | Cl | H | $CH_3$ | H | H | - | - | 0 | 2 | 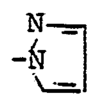 | 98-100 °C |
| 30 | Cl | H | H | $CH_3$ | H | H | - | - | 0 | 2 | 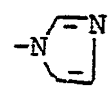 | 174 °C |
| 31 | Cl | Cl | H | $CH_3$ | H | H | $CH_3$ | $CH_3$ | 1 | 1 | 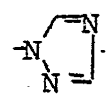 | |
| 32 | Cl | H | H | $CH_3$ | H | H | $CH_3$ | $CH_3$ | 1 | 1 | 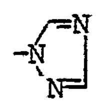 | |

In den folgenden Verwendungsbeispielen wird die nachstehend angegebene Verbindung als Vergleichssubstanz eingesetzt:

(A) = 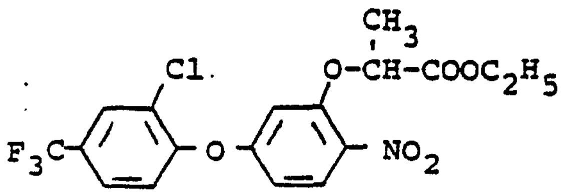

(bekannt aus DE-OS 2 311 638 bzw. US-PS 4 093 446)
α-(5-(2-Chlor-4-trifluormethyl-phenoxy)-2-nitro-phenoxy)-propionsäureethylester

0073880

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % keine Wirkung (wie unbehandelte Kontrolle)
100 % totale Vernichtung

In diesem Test zeigen die erfindungsgemäßen Wirkstoffe (1) und (2) eine bessere selektive herbizide Wirksamkeit als die Vergleichssubstanz (A).

Beispiel B

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
+ leichtes Austrocknen der Blätter, geringer Blattfall
++ starkes Austrocknen der Blätter, starker Blattfall
+++ sehr starkes Austrocknen der Blätter, sehr starker Blattfall

In diesem Test zeigen die erfindungsgemäßen Wirkstoffe (1) und (2) eine sehr starke Wirksamkeit.

Patentansprüche

1. Substituierte Phenoxyphenoxyalkancarbonsäure-azolylalkylester der Formel

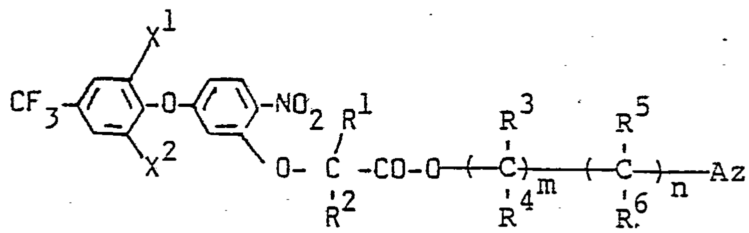

(I)

in welcher

$X^1$ und $X^2$ unabhängig voneinander für Wasserstoff oder Halogen stehen,

$R^1$, $R^2$, $R^3$, $R^4$, $R^5$ und $R^6$ unabhängig voneinander für Wasserstoff oder Alkyl mit 1 bis 4 Kohlenstoffatomen stehen,

m für 1 oder 2 steht,

n für 0 oder 1 steht und

Az für einen gegebenenfalls substituierten Azolylrest steht,

sowie deren Säureadditions-Salze, Metallsalz-Komplexe und quaternäre Alkylierungsmittel-Additionssalze.

2. Substituierte Phenoxyphenoxyalkancarbonsäure-azolylalkylester der Formel (I), in denen

$X^1$ für Chlor steht,
$X^2$ für Wasserstoff oder Chlor steht,

$R^1$,$R^2$,$R^3$, $R^4$,$R^5$ und $R^6$ unabhängig voneinander für Wasserstoff oder Methyl stehen,
m für 1 oder 2 steht,
n für 0 oder 1 steht und

Az für einen über ein Ring-Stickstoffatom gebundenen Pyrazolyl-, Imidazolyl-, 1,2,4-Triazolyl-, 1,2,3-Triazolyl- oder 1,3,4-Triazolyl-Rest steht, wobei jeder dieser Azolyl-Reste ein- oder mehrfach, gleichartig oder verschieden substituiert sein kann durch Fluor, Chlor, Brom, Jod, Alkyl mit 1 bis 4 Kohlenstoffatomen, welches seinerseits gegebenenfalls durch Fluor, Chlor, Cyano, Alkoxy mit 1 bis 4 Kohlenstoffatomen, Alkylthio mit 1 bis 4 Kohlenstoffatomen, Alkylsulfinyl mit 1 bis 4 Kohlenstoffatomen, Alkylsulfonyl mit 1 bis 4 Kohlenstoffatomen und/oder Alkoxycarbonyl mit 1 bis 4 Kohlenstoffatomen in der Alkoxygruppe substituiert ist, Alkoxy mit 1 bis 4 Kohlenstoffatomen, Alkylthio mit 1 bis 4 Kohlenstoffatomen, Cyano, Carboxy, Alkoxycarbonyl mit 1 bis 4 Kohlenstoffatomen in der Alkoxygruppe, Alkylcarbonyl mit 1 bis 4 Kohlenstoffatomen in der Alkylgruppe und/oder Phenyl, welches seinerseits gegebenenfalls durch Halogen, Alkyl mit 1 bis 4 Kohlenstoffatomen, Trifluormethyl, Cyano, Nitro und/oder Alkoxy mit 1 bis 4 Kohlenstoffatomen substituiert ist,

sowie deren Säureadditions-Salze, Metallsalz-Komplexe und quaternäre Alkylierungsmittel-Additionssalze.

3. Verfahren zur Herstellung von substituierten Phenoxyphenoxyalkancarbonsäure-azolylalkylestern der Formel

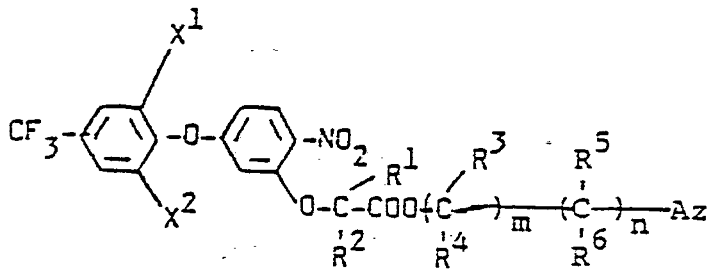

(I)

in welcher

$X^1$ und $X^2$ unabhängig voneinander für Wasserstoff oder Halogen stehen,

$R^1$, $R^2$, $R^3$, $R^4$, $R^5$ und $R^6$ unabhängig voneinander für Wasserstoff oder Alkyl mit 1 bis 4 Kohlenstoffatomen stehen,

m für 1 oder 2 steht,

n für 0 oder 1 steht und

Az für einen gegebenenfalls substituierten Azolylrest steht,

sowie deren Säureadditions-Salzen Metallsalz-Komplexen und quaternären Alkylierungsmittel-Additionssalzen, dadurch gekennzeichnet, daß man

(a) Phenoxyphenoxyalkancarbonsäurechloride der Formel

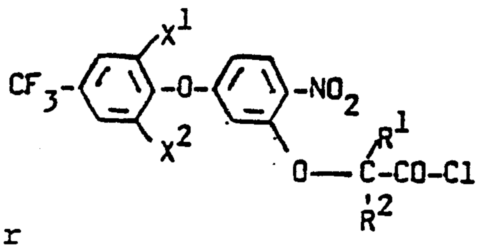

(II)

in welcher

$X^1, X^2, R^1$ und $R^2$ die oben angegebene Bedeutung haben,

mit Hydroxyalkylazolen der Formel

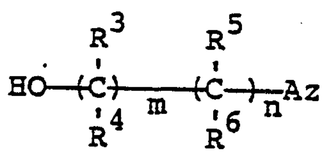

(III)

in welcher

$R^3, R^4, R^5, R^6$, Az, m und n die oben angegebene Bedeutung haben,

gegebenenfalls in Gegenwart eines Säureakzeptors und gegebenenfalls in Gegenwart eines Verdünnungsmittels umsetzt, oder daß man

(b) Phenoxyphenole der Formel

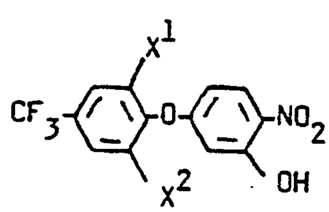

(IV)

in welcher

$X^1$ und $X^2$ die oben angegebene Bedeutung haben

mit Halogenalkancarbonsäure-azolylalkylestern der Formel

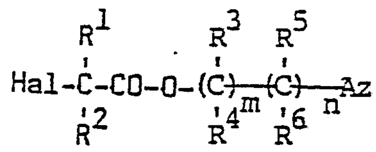

(V)

in welcher

$R^1$, $R^2$, $R^3$, $R^4$, $R^5$, $R^6$, Az, m und n die oben angegebene Bedeutung haben und

Hal für Chlor oder Brom steht,

gegebenenfalls in Gegenwart eines Säureakzeptors und gegebenenfalls in Gegenwart eines Verdünnungsmittels umsetzt, oder daß man

(c) Phenoxyphenoxyalkancarbonsäure-azolylalkylester der Formel

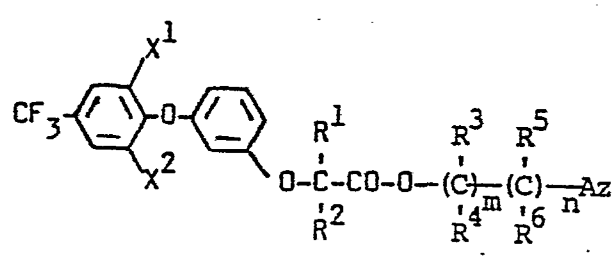

(VI)

in welcher

$X^1, X^2, R^1, R^2, R^3, R^4, R^5, R^6$, Az, m und n die oben angegebene Beswutung haben,

mit Salpetersäure gegebenenfalls in Gegenwart eines Katalysators und gegebenenfalls in Gegenwart eines Verdünnungsmittels umsetzt,

und gegebenenfalls anschließend an die so erhaltenen Phenoxyphenoxyalkancarbonsäure-azolylalkylester der Formel (I) eine Säure, ein Metallsalz oder ein Alkylierungsmittel addiert.

4. Herbizide und pflanzenwuchsregulierende Mittel, gekennzeichnet durch einen Gehalt an mindestens einem substituierten Phenoxyphenoxyalkancarbonsäure-azolylalkylester der Formel (I) bzw. einem Säureadditions-Salz, Metallsalz-Komplex oder quaternären Alkylierungsmittel-Additionssalz von einem substituierten Phenoxyphenoxyalkancarbonsäure-azolylalkylester der Formel (I).

5. Verwendung von substituierten Phenoxyphenoxyalkancarbonsäure-azolylalkylestern der Formel (I) bzw. von deren Säureadditions-Salzen, Metallsalz-Komplexen und quaternären Alkylierungsmittel-Additionssalzen zur Bekämpfung von Unkräutern sowie zur Regulierung des Pflanzenwachstums.

6. Verfahren zur Herstellung von herbiziden bzw. pflanzenwuchsregulierenden Mitteln, dadurch gekenn-

zeichnet, daß man substituierte Phenoxyphenoxy-alkancarbonsäure-azolylalkylester der Formel (I) bzw. deren Säureadditions-Salze, Metallsalz-Komplexe oder quaternäre Alkylierungsmittel-Additionssalze mit Streckmitteln und/oder oberflächenaktiven Stoffen vermischt.

7. Halogenalkancarbonsäure-azolylalkylester der Formel

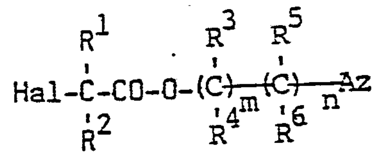

(V)

in welcher

$R^1$, $R^2$, $R^3$, $R^4$, $R^5$ und $R^6$ unabhängig voneinander für Wasserstoff oder Alkyl mit 1 bis 4 Kohlenstoffatomen stehen,

m für 1 oder 2 steht,

n für 0 oder 1 steht,

Az für einen gegenenfalls substituierten Azolylrest steht und

Hal für Chlor oder Brom steht.

8. Verfahren zur Herstellung von Halogenalkancarbonsäure-azolylalkylestern der Formel

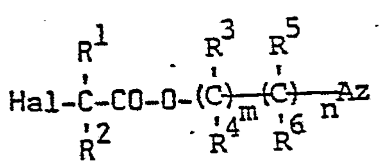

(V)

in welcher

$R^1$, $R^2$, $R^3$, $R^4$, $R^5$ und $R^6$ unabhängig voneinander für Wasserstoff oder Alkyl mit 1 bis 4 Kohlenstoffatomen stehen,

m für 1 oder 2 steht,

n für 0 oder 1 steht,

Az für einen gegebenenfalls substituierten Azolylrest steht und

Hal für Chlor oder Brom steht,

dadurch gekennzeichnet, daß man Halogencarbonsäurehalogenide der Formel

$$\text{Hal}-\overset{R^1}{\underset{R^2}{C}}-\text{CO}-\text{Hal}' \qquad \text{(VII)}$$

in welcher

$R^1$ und $R^2$ die oben angegebene Bedeutung haben und Hal sowie Hal' für Chlor oder Brom stehen,

mit Hydroxyalkylazolen der Formel

$$\text{HO}-\left(\overset{R^3}{\underset{R^4}{C}}\right)_m-\left(\overset{R^5}{\underset{R^6}{C}}\right)_n-\text{Az} \qquad \text{(III)}$$

in welcher

$R^3$, $R^4$, $R^5$, $R^6$, Az, m und n die oben angegebene Bedeutung haben,

gegebenenfalls in Gegenwart eines Säureakzeptors und gegebenenfalls in Gegenwart eines Verdünnungsmittels umsetzt.

9. Phenoxyphenoxyalkancarbonsäure-azolylalkylester der Formel

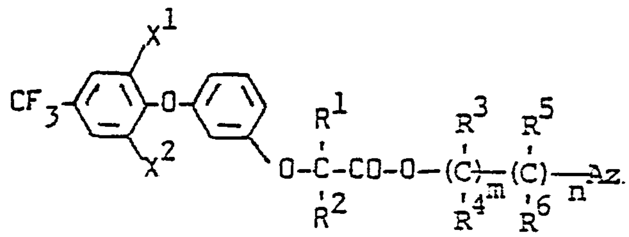

(VI)

in welcher

$X^1$ und $X^2$ unabhängig voneinander für Wasserstoff oder Halogen stehen,

$R^1$, $R^2$, $R^3$, $R^4$, $R^5$ und $R^6$ unabhängig voneinander für Wasserstoff oder Alkyl mit 1 bis 4 Kohlenstoffatomen stehen,

m für 1 oder 2 steht,

n für 0 oder 1 steht und

Az für einen gegebenenfalls substituierten Azolylrest steht.

10. Verfahren zur Herstellung von Phenoxyphenoxyalkancarbonsäure-azolylalkylestern der Formel

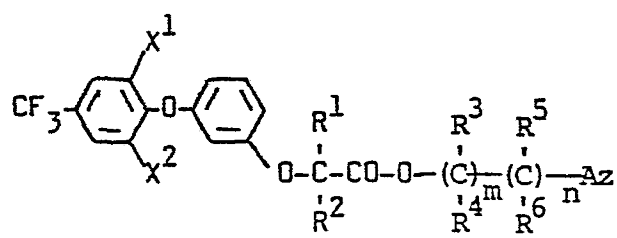

(VI)

in welcher

$X^1$ und $X^2$ unabhängig voneinander für Wasserstoff oder Halogen stehen,

$R^1$, $R^2$, $R^3$, $R^4$, $R^5$ und $R^6$ unabhängig voneinander für Wasserstoff oder Alkyl mit 1 bis 4 Kohlenstoffatomen stehen,

m für 1 oder 2 steht,

n für 0 oder 1 steht und

Az für einen gegebenenfalls substituierten Azolylrest steht,

dadurch gekennzeichnet, daß man Phenoxyphenole der Formel

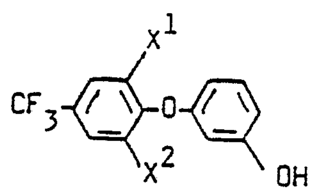

(VIII)

in welcher

$X^1$ und $X^2$ die oben angegebene Bedeutung haben, mit Halogenalkancarbonsäure-azolylalkylestern der Formel

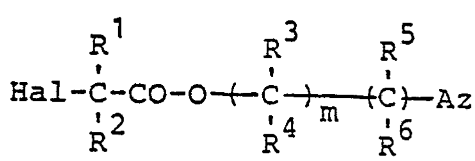

(V)

in welcher

$R^1$, $R^2$, $R^3$, $R^4$, $R^5$, $R^6$, Az, m, n und Hal die oben angegebene Bedeutung haben,

gegebenenfalls in Gegenwart eines Säureakzeptors und gegebenenfalls in Gegenwart eines Verdünnungsmittels umsetzt.